# EUROPEAN PATENT APPLICATION

(11) **EP 4 549 573 A1**
(43) Date of publication of application: **07.05.2025**
(21) Application number: 23852186.8
(22) Date of filing: 26.04.2023
(51) Int. Cl.: C12N 15/62, A61K 31/7088, A61K 35/76, A61K 38/17, A61K 48/00, A61P 43/00, C07K 2/00, C07K 19/00, C12N 15/63

(54) **PEPTIDE LINKER AND LOCALIZATION METHOD FOR LOCALIZING TRANSMEMBRANE PROTEIN TO TARGET ORGANELLE, AND LOCALIZING FUSION PROTEIN**

(30) Priority: 12.08.2022 JP 2022128808
(71) Applicant: Hamamatsu Photonics K.K., Hamamatsu-shi, Shizuoka 435-8558 (JP)
(72) Inventor: SUZUKI Kazuya, Hamamatsu-shi, Shizuoka 435-8558 (JP)
(74) Representative: Grünecker Patent- und Rechtsanwälte PartG mbB
(86) International application number: PCT/JP2023/016521
(87) International publication number: WO 2024/034200

(57) **Abstract**

Disclosed are a highly rigid peptide linker inserted between a transmembrane protein and an organelle transport signal, a fusion protein containing the same, and a localization method that includes inserting a peptide linker.

## Description

### Technical Field

The present invention relates to a peptide linker and a localization method for localizing a transmembrane protein to a target organelle and a localizing fusion protein.

### Background Art

A transmembrane protein is a kind of membrane protein localized in a cell membrane or an organelle membrane. In a state in which the transmembrane protein is localized in a membrane, a hydrophobic transmembrane region exists so as to penetrate a lipid bilayer membrane, and other regions appear outside the lipid bilayer membrane. As a major transmembrane protein, a channel and a pump, which are transmembrane proteins (transporters) that transport specific substances to the opposite side of a lipid bilayer membrane, a cell membrane receptor, which is a transmembrane protein that transmits a specific input from the outside of a cell as an intracellular signal, a membrane-localized enzyme, and the like are known, and perform various functions of a biological membrane.

Inability to perform biosynthesis of a protein essential for a biological function is an event that may bring about a serious risk to life, and in particular, in a case where biosynthesis of a protein having an appropriate structure cannot be performed congenitally due to a genetic disease or the like, radical cure is extremely difficult. As a method of treating such a congenital genetic disease, a treatment (gene therapy) in which a gene is externally introduced into human somatic cells is expected, but there are few practical examples.

A mitochondrial disease, which is one of congenital genetic diseases, is a generic term for diseases caused by dysfunctions of mitochondria, and is a disease that causes various serious symptoms including deterioration in muscle strength and delayed development progress. In eukaryotic cells, mitochondria are the only organelles excluding nuclei that have DNA unique to an organelle called mitochondrial DNA (mtDNA) and can express a protein in the organelle. Mitochondrial proteins expressed by mitochondrial DNA are essential for exertion of functions of mitochondria. In a mitochondrial disease, abnormalities in protein expression by mitochondrial DNA are observed.

Since the influence of substance transport, signal transmission, and the like by a transmembrane protein on cells significantly depends on a location at which the transmembrane protein is localized, localization control thereof has been attempted. For example, Non Patent Literature 1 discloses a technique for improving localization to mitochondria by overlapping mitochondria targeting signals at the N-terminus of subunit 9 of ATP synthase which is the transmembrane protein. In addition, Non Patent Literature 2 discloses a technique for localizing ATP6, which is a five times transmembrane-type transmembrane protein, in mitochondria by adding, to a terminus of 5', a mitochondria targeting signal region of protein SOD2 in which mRNA itself exists in mitochondria and by adding a 3' untranslated region of SOD2 to a terminus of 3'.

### Citation List

### Non Patent Literature

Non Patent Literature 1: Galanis M. et al., "Duplication of leader sequence for protein targeting to mitochondria leads to increased import efficiency", FEBS Letters, 282, 425-430 (1991).
Non Patent Literature 2: Kaltimbacher V. et al., "mRNA localization to the mitochondrial surface allows the efficient translocation inside the organelle of a nuclear recoded ATP6 protein", RNA,12, 1408-1417 (2006).
Non Patent Literature 3: Miyazaki E. et al., "Switching the Sorting Mode of Membrane Proteins from Cotranslational Endoplasmic Reticulum Targeting to Posttranslational Mitochondrial Import", Mol. Biol. Cell, 16, 1788-1799 (2005).

### Summary of Invention

### Technical Problem

It is expected to achieve, through a technique for localizing a transmembrane protein in a specific organelle, medical application and biochemical application including application to gene therapy of congenital genetic diseases. For example, mitochondria are organelles composed of a double membrane, and most of mitochondrial proteins are transmembrane proteins localized in a membrane. Therefore, it can be expected to achieve, through a technique for localizing the transmembrane proteins in mitochondria, medical application to treatment of a mitochondrial disease by supplementation of a mitochondrial protein.

However, unlike a case in which a normal protein (for example, a cytosolic localization protein) is localized in a target organelle, it is known that, even if an organelle localization signal is imparted to a transmembrane protein, the transmembrane protein is aggregated between molecules or migrates to endoplasmic reticulum due to its high hydrophobicity, and thus the transmembrane protein cannot be smoothly localized in the target organelle in many cases.

It is therefore an object of the present invention to provide a method of localizing a transmembrane protein to a target organelle.

### Solution to Problem

As a result of intensive studies by the present inventors, it has been found that, when an organelle localization signal is imparted to a terminus of a transmembrane protein via a proline-rich peptide linker known as a linker having high rigidity or a peptide linker having an α-helix as a secondary structure (hereinafter, these are also collectively referred to as "rigid linkers"), the transmembrane protein is localized to an organelle corresponding to the organelle localization signal, thereby completing the present invention.

That is, the present invention relates to the following [1] to [15].
[1] A peptide linker for localizing a transmembrane protein to a target organelle, the peptide linker being inserted between the transmembrane protein and an organelle localization signal corresponding to the target organelle, wherein the number of proline residues contained in the peptide linker exceeds 40% of the number of all amino acid residues contained in the peptide linker, and the number of amino acid residues constituting the peptide linker is 60 residues or more.
[2] A peptide linker for localizing a transmembrane protein to a target organelle, the peptide linker being inserted between the transmembrane protein and an organelle localization signal corresponding to the target organelle, wherein the peptide linker is formed of a peptide of which the secondary structure is an α-helix, and the number of amino acid residues constituting the peptide linker is 70 residues or more.
[3] A fusion protein comprising: a transmembrane protein; an organelle localization signal for localization to a target organelle; and a peptide linker inserted between the transmembrane protein and the organelle localization signal, wherein the number of proline residues contained in the peptide linker exceeds 40% of the number of all amino acid residues contained in the peptide linker, and the number of amino acid residues constituting the peptide linker is 60 residues or more.
[4] A fusion protein comprising: a transmembrane protein; an organelle localization signal for localization to a target organelle; and a peptide linker inserted between the transmembrane protein and the organelle localization signal, wherein the peptide linker is formed of a peptide of which the secondary structure is an α-helix, and the number of amino acid residues constituting the peptide linker is 70 residues or more.
[5] A method of localizing a transmembrane protein to a target organelle, the method including inserting a peptide linker between the transmembrane protein and an organelle localization signal for localization to the target organelle, wherein the number of proline residues contained in the peptide linker exceeds 40% of the number of all amino acid residues contained in the peptide linker, and the number of amino acid residues constituting the peptide linker is 60 residues or more.
[6] A method of localizing a transmembrane protein to a target organelle, the method including inserting a peptide linker between the transmembrane protein and an organelle localization signal for localization to the target organelle, wherein the peptide linker is formed of a peptide of which the secondary structure is an α-helix, and the number of amino acid residues constituting the peptide linker is 70 residues or more.
   [6-1] A nucleic acid comprising a sequence encoding the peptide linker according to [1] or [2].
[7] A nucleic acid encoding the fusion protein according to [3] or [4].
[8] A vector expressing the fusion protein according to [3] or [4].
[9] The peptide linker, the fusion protein, the localization method, the nucleic acid, or the vector according to any one of [1] to [8], wherein the target organelle is selected from the group consisting of nucleus, mitochondria, chloroplast, endoplasmic reticulum, and peroxisome.
[10] The peptide linker, the fusion protein, the method, the nucleic acid, or the vector according to any one of [1] to [8], wherein the target organelle is nucleus or mitochondria.
[11] The peptide linker, the fusion protein, the method, the nucleic acid, or the vector according to any one of [1] to [8], wherein the target organelle is mitochondria.
[12] A therapeutic agent for a mitochondrial disease, the therapeutic agent comprising the fusion protein according to [3] or [4] or the vector according to [8], wherein the target organelle is mitochondria.
[13] A method of treating a mitochondrial disease, wherein the target organelle is mitochondria, and the fusion protein according to [3] or [4] or the vector according to [8] is administered to a patient.
[14] The fusion protein according to [3] or [4] or the vector according to [8] for use in the treatment of a mitochondrial disease, wherein the target organelle is mitochondria.
[15] Use of the fusion protein according to [3] or [4] or the vector according to [8] in the manufacture of a therapeutic agent for a mitochondrial disease, wherein the target organelle is mitochondria.

### Advantageous Effects of Invention

According to the present invention, it is possible to provide a peptide linker and a localization method of localizing a transmembrane protein in a target organelle, and a localized fusion protein.

### Brief Description of Drawings

FIG. 1 is a diagram showing an outline of a structure of a fusion protein according to a peptide linker, a fusion protein, and a localization method of the present invention.
FIG. 2 is a diagram showing a schematic diagram of a fusion protein in which a flexible linker is inserted between a transmembrane protein and an organelle localization signal. Since the peptide linker is flexible, it is presumed that the organelle localization signal and the transmembrane protein cause an intramolecular hydrophobic interaction and cannot be localized in a target organelle.
FIG. 3 is a diagram showing a schematic diagram of a fusion protein in which a rigid linker is inserted between the transmembrane protein and the organelle localization signal. Since the peptide linker is rigid, it is presumed that the organelle localization signal and the transmembrane protein do not cause the intramolecular hydrophobic interaction and can be localized in the target organelle.
FIG. 4 is a diagram showing a mechanism of treatment of a mitochondrial disease by replacement therapy.
FIG. 5 is a diagram showing an outline of a nucleic acid corresponding to a translation region of a plasmid used for expressing a protein in a cell in Examples.
FIG. 6 is a diagram showing a relationship between the number of amino acid residues contained in a proline linker and a correlation coefficient in co-localization analysis in Example 1.
FIG. 7 is a diagram showing a representative fluorescence image in a group in which the number of amino acid residues contained in the proline linker is 0 in Example 1.
FIG. 8 is a diagram showing a representative fluorescence image including a plurality of cells in a group in which the number of amino acid residues contained in the proline linker is 0 in Example 1.
FIG. 9 is a diagram showing a representative fluorescence image in a group in which the number of amino acid residues contained in the proline linker is 70, 80, 90, 100, 150, and 200 residues in Example 1.
FIG. 10 is a diagram showing a relationship between the proportion of proline residues in amino acid residues contained in the proline linker and a correlation coefficient in co-localization analysis in Example 2.
FIG. 11 is a diagram showing a representative fluorescence image in a group in which the proportion of proline residues in the amino acid residues contained in the proline linker is 30% in Example 2.
FIG. 12 is a diagram showing a representative fluorescence image containing a plurality of cells in a group in which the proportion of proline residues in the amino acid residues contained in the proline linker is 30% in Example 2.
FIG. 13 is a diagram showing a relationship between the number of amino acid residues contained in an α-helix linker and a correlation coefficient in co-localization analysis in Example 3.
FIG. 14 is a diagram showing a representative fluorescence image in a group in which the number of amino acid residues contained in the α-helix linker is 300 residues in Example 3.
FIG. 15 is a diagram showing a representative fluorescence image in a group in which the number of amino acid residues contained in the α-helix linker is 200 residues in Example 3.

### Description of Embodiments

One aspect of the present invention is a peptide linker that is used for localizing a transmembrane protein in a target organelle and is inserted between the transmembrane protein and an organelle localization signal corresponding to the target organelle.

Another aspect of the present invention is a fusion protein comprising a transmembrane protein, an organelle localization signal, and a peptide linker inserted between the transmembrane protein and the organelle localization signal.

Another aspect of the present invention is a method (hereinafter, the method is also referred to as a "localization method") for localizing a transmembrane protein to an organelle corresponding to an organelle localization signal, in which the method includes inserting a peptide linker between the transmembrane protein and the organelle localization signal.

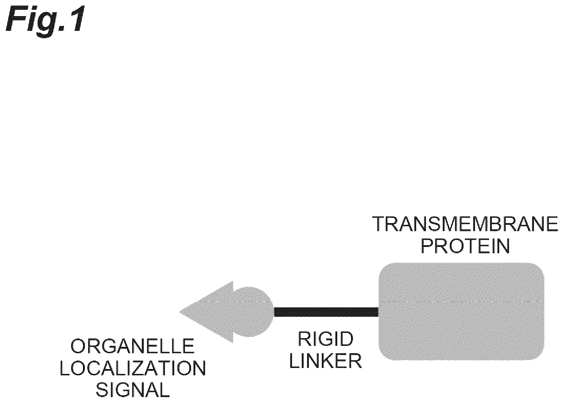

As shown in FIG. 1, a fusion protein according to a peptide linker, a fusion protein, and a localization method of the present invention includes at least a transmembrane protein, an organelle localization signal, and a peptide linker inserted between the transmembrane protein and the organelle localization signal, and the peptide linker is a rigid linker.

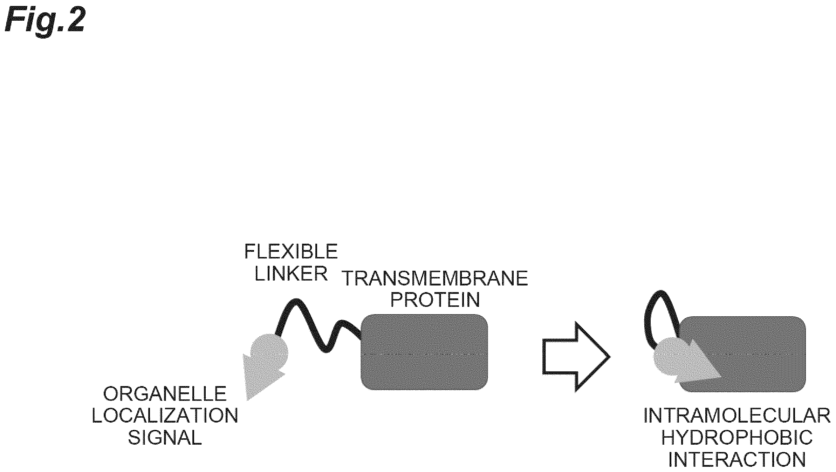

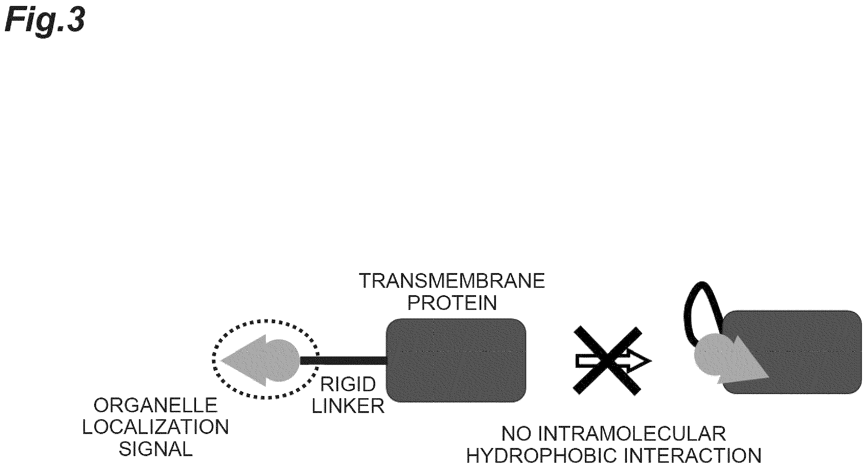

Without wishing to be bound by any theory, a mechanism by which the fusion protein according to the peptide linker, the fusion protein, and the localization method of the present invention can be localized in a target organelle is presumed as follows. When the peptide linker inserted between the transmembrane protein and the organelle localization signal is a linker having low rigidity (hereinafter, also referred to as a flexible linker), since the transmembrane protein has high hydrophobicity, as shown in FIG. 2, a hydrophobic region of the transmembrane protein and the organelle localization signal has a hydrophobic interaction in a molecule. Then, since a recognition mechanism of the organelle localization signal such as a signal recognition particle (SRP) or a pre-sequence receptor cannot recognize the organelle localization signal, it is presumed that the fusion protein cannot be localized in the target organelle. On the other hand, when the peptide linker inserted between the transmembrane protein and the organelle localization signal is the rigid linker according to the peptide linker, the fusion protein, and the localization method of the present invention, the rigid linker is not bent, and the transmembrane protein and the organelle localization signal do not undergo a hydrophobic interaction in the molecule. Therefore, as shown in FIG. 3, the organelle localization signal can exist in a free state from the transmembrane protein. Then, since the recognition mechanism of the organelle localization signal such as the signal recognition particle (SRP) or the pre-sequence receptor can recognize the organelle localization signal, it is presumed that the fusion protein can be localized in the target organelle.

### (1) Transmembrane Protein

A transmembrane protein is a membrane protein that exists through a lipid bilayer membrane of a biological membrane, and is a major component of the biological membrane. The transmembrane protein exists as a pump, a transporter, an ion channel, a receptor, an enzyme, a membrane structural protein, or the like, and plays various functions of the biological membrane. One or a plurality of hydrophobic regions (hereinafter, also referred to as "transmembrane domains") exist in the molecule of the transmembrane protein and are fixed to the biological membrane by hydrophobic interaction, and the other regions appear outside the lipid bilayer membrane. In general, the hydrophobic region is composed of about 20 highly hydrophobic amino acids, and highly hydrophobic amino acids such as amino acids having charges exist at both ends thereof.

The transmembrane protein according to the peptide linker, the fusion protein, and the localization method of the present invention may penetrate the lipid bilayer membrane once or more when transmembrane protein is localized in the cell membrane or the organelle membrane, and may be, for example, a once transmembrane protein or a multiple transmembrane protein. In particular, the once transmembrane protein is classified into a type I in which the N-terminus side is exposed in the endoplasmic reticulum lumen in a state of being inserted into the endoplasmic reticulum membrane in a translation process and a type II in which the C-terminus side is exposed, but the once transmembrane protein according to the peptide linker, the fusion protein, and the localization method of the present invention may be the type I or the type II. In addition, a secondary structure of the transmembrane domain of the transmembrane protein is not particularly limited, and may be, for example, an α-helix type or a β-sheet type.

An origin of the transmembrane protein according to the peptide linker, the fusion protein, and the localization method of the present invention is not particularly limited, and the origin may be a transmembrane protein existing in nature, may be a transmembrane protein having a partial amino acid residue substituted or deleted as long as a function of a protein existing in nature is not impaired, or may be an artificially produced transmembrane protein. In addition, when the transmembrane protein is derived from a eukaryotic cell, localization under physiological conditions is not particularly limited, and may be, for example, a protein localized in a cell membrane, or may be a protein localized in a membrane of an organelle such as a nucleus, a mitochondrion, a chloroplast, an endoplasmic reticulum, a Golgi body, a secretory granule, a secretory vesicle, a lysosome, a phagosome, an endosome, a vacuole, or a peroxisome. In addition, the transmembrane protein may be obtained by substituting or deleting a part or all of the amino acid sequence corresponding to the organelle localization signal from the transmembrane protein localized in the organelle.

Examples of the transmembrane protein according to the present invention include aquaporin, a CLC family ion channel, bestrophin, a Cys loop-type ligand-dependent ion channel, an ion channel-type glutamic acid receptor, an ion channel-type ATP receptor, a voltage-dependent potassium channel, a cyclic nucleotide-dependent channel, a calcium-activated potassium channel, a TRP channel, a sodium channel, a calcium channel, a CatSper channel, a 2-pore potassium channel, an inward rectifying potassium channel, a voltage-dependent anion channel (VDAC), an ABC transporter, a P-type ATPase, a V-type ATPase, an SLC35 gene family transporter, an SLC family transporter, an APC superfamily transporter, a mitochondrial carrier, an MFS-type transporter, sideroflexin, a vesicle-associated membrane protein (VAMP), a rhodopsin phosphodiesterase, an adhesion G protein-coupled receptor, a cecretin receptor, a metabolic glutamate receptor, Frizzled/Taste2 receptor, EGF receptor-activated kinase, FGF receptor-activated kinase, ephrin receptor-activated kinase, serine/threonine kinase receptor, AXL-activated kinase, insulin receptor, receptor guanylate cyclase, PDGF receptor, TNF/NGF receptor, integrin, receptor tyrosine phosphatase, prexin, Notch, selectin, contactin, NADH-ubiquinone oxidative reductase, cytochrome c oxidase, flavin-containing monooxygenase, nonspecific monooxygenase, cytochrome P450, acyl group transfer enzyme, glycosyltransferase, phosphate transfer enzyme, sulfate transfer enzyme, non-receptor tyrosine phosphorylase, phosphate diester hydrolase, membrane-localized peptidase, nucleoside diphosphatase, adenylate cyclase, SAM complex constituent proteins, TOM complex constituent proteins, TIM complex constituent proteins, TIM complex constituent proteins, mitofusin, and ERMES complex constituent proteins.

### (2) Organelle Localization Signal

In the present specification, an organelle localization signal refers to a peptide having a property of localizing a protein to an organelle (target organelle) corresponding to the organelle localization signal by imparting the organelle localization signal to the protein. In general, a protein existing as a free state in a cell represented by a cytoplasmic localization protein is likely to be localized in a target organelle by imparting an organelle localization signal, whereas a protein exhibiting strong localization to a specific organelle in a physiological state is less likely to be localized by the organelle localization signal.

The organelle localization signal according to the peptide linker, the fusion protein, and the localization method of the present invention is not particularly limited as long as the protein can be localized in the target organelle by being imparted to the protein, and may be one existing in nature, one in which some amino acid residues are substituted or deleted as long as a function of the organelle localization signal existing in nature is not impaired, or one that is artificially created. When the organelle localization signal exists in nature, the organelle localization signal may be the full length or a part of the protein localized in the target organelle, but from the viewpoint of avoiding inhibition of the function of the transmembrane protein, the organelle localization signal is preferably a part of the protein localized in the target organelle.

The amino acid sequence of the protein localized in the target organelle can be obtained from a known protein database, for example, the protein database of the National Center for Biotechnology Information (NCBI).

Examples of the target organelle include nuclei, mitochondria, chloroplasts, endoplasmic reticulum, Golgi apparatus, secretory granules, secretory vesicles, lysosomes, phagosomes, endosomes, vacuoles, and peroxisomes. From the viewpoint of controlling protein dynamics or energy production in cells, nuclei, mitochondria, chloroplasts, endoplasmic reticulum, or peroxisomes are preferable, from the same viewpoint, nuclei or mitochondria are more preferable, and from the viewpoint of controlling functions of the cells, mitochondria are particularly preferable.

When the target organelle is a mitochondrion, the organelle localization signal is generally imparted to N-terminus. As a mitochondria targeting signal (also referred to as MTS), a peptide described in a known literature (refer to, for example, Gunnar von HEIJNE et al., "EUROPEAN JOURNAL OF BIOCHEMISTRY", 1989, vol. 180, pages 535 to 545) can be used. From the viewpoint of enhancing localization to mitochondria, the number of amino acid residues constituting the mitochondria targeting signal is preferably 15 residues or more and 300 residues or less, more preferably 16 residues or more and 250 residues or less, still more preferably 17 residues or more and 200 residues or less, and still further more preferably 18 residues or more and 160 residues or less. In addition, from the viewpoint of enhancing localization to mitochondria by interaction with the Tom complex, the number of leucine residues contained in the mitochondria targeting signal may be 10% or more of the number of all amino acid residues contained in the organelle localization signal. From the same viewpoint, the number of arginine residues contained in the mitochondria targeting signal may be 10% or more of the number of all amino acid residues contained in the organelle localization signal. An example of the mitochondria targeting signal can include, for example, a peptide having an amino acid sequence shown in the following SEQ ID NOs: 1 to 3, which is imparted to the N-terminus of the protein.
(a)MLSLRQSIRFFKPATRTLCSSRYL(SEQ ID NO: 1)
(b) 
MRAPSARALLLIPRRGPAVRAWAPAVSSRIWLASEWTP
LVRAWTSLIHKPGSGLRFPAPLSGLPGGVGQWATSSGARRCWVL
AGPRAAHPLFARLQGAAATGVRDLGNDSQRRPAATGRSEVWKL
LGLVRPERGRLSAAV(SEQ ID NO: 2)
(c):

MALLRAAVSELRRRGRGALTPLPALSSLLSSLSPRSPAS
TRPEPNNPHADRRHVIALRRCPPLPASAVLAPELLHARGLLPRH
WSHASPLSTSSSSSRPADKAQLTWVDKWIPEAARPY(SEQ ID NO:
3)

When the target organelle is a nucleus, in general, since the organelle localization signal can be imparted to a portion other than the terminus of the peptide, the nuclear localization signal (also referred to as NLS) may be imparted to the N-terminus or the C-terminus, and a peptide chain may be imparted to the further N-terminus side of the nuclear localization signal imparted to the N-terminus or the further C-terminus side of the nuclear localization signal imparted to the C-terminus. As the nuclear localization signal, a peptide described in a known literature (for example, Shunichi Kosugi et al., "JOURNAL OF BIOLOGICAL CHEMISTRY", 2009, vol. 284, p478 to 485 and Allison Lange et al., "JOURNAL OF BIOLOGICAL CHEMISTRY", 2007, vol. 282, pages 5101 to 5105) can be used. In the nuclear localization signal, there are a mono-node type in which a signal sequence is a region of 1 and a di-node type in which a signal sequence is a region of 2 in the peptide, but the nuclear localization signal according to the peptide linker, the fusion protein, and the localization method of the present invention may be a mono-node type or a di-node type. The number of amino acid residues constituting the nuclear localization signal is, for example, 20 residues or less, and may be 18 residues or less, or 16 residues or less. In addition, from the viewpoint of enhancing nuclear localization, the proportion of the total number of lysine residues and arginine residues to the number of amino acid residues constituting the nuclear localization signal may be 20% or more. As an example of the nuclear localization signal, a peptide having an amino acid sequence shown in the following SEQ ID NO: 4 can be mentioned.

### (a)PKKKRRV(SEQ ID NO: 4)

When the target organelle is a peroxisome, a peroxisome targeting signal (PTS), which is an organelle localization signal, is classified into PTS1 provided on the C-terminus side and PTS2 provided on the N-terminus side, but the PTS according to the peptide linker, the fusion protein, and the localization method of the present invention may be PTS1 or PTS2. Examples of PTS1 and PTS2 include peptides shown below. Hereinafter, X in the described amino acid sequence represents any amino acid unless otherwise specified.
(a) SKL
(b) RLXXXXXHL

When the target organelle is a chloroplast, the organelle localization signal is generally provided at the N-terminus. As a chloroplast localization signal, a peptide described in a known literature (refer to, for example, Gunnar von HEIJNE et al., "EUROPEAN JOURNAL OF BIOCHEMISTRY", 1989, vol. 180, pages 535 to 545) can be used, and a chloroplast localization signal peptide (transit peptide) of a small subunit of RubisCo (rubisco, ribulose bisphosphate carboxylase/oxygenase, ribulose diphosphate carboxyltransferase/oxygenase) of any plant species can also be used. From the viewpoint of enhancing localization to chloroplasts, the number of amino acid residues constituting the chloroplast localization signal is preferably 30 or more and 100 or less.

### (3) Peptide Linker

The peptide linker according to the peptide linker, the fusion protein, and the localization method of the present invention is a peptide linker composed of α amino acids (linker formed by peptide-bonded amino acids), and is a proline-rich peptide linker (hereinafter, also referred to as a proline linker) known as a linker having high rigidity or a peptide linker having a secondary structure as an α helix (hereinafter, also referred to as an α-helix linker). In the present specification, proline is encompassed by the α amino acids.

When the peptide linker according to the peptide linker, the fusion protein, and the localization method of the present invention is the proline linker, from the viewpoint of enhancing the localization of the fusion protein to the target organelle by inhibiting an intramolecular hydrophobic interaction, the number of proline residues contained in the peptide linker may be 30% or more, preferably 40% or more, more preferably more than 40%, and still more preferably 50% or more of the number of all amino acid residues contained in the peptide linker. From the same viewpoint, for amino acid residues other than the proline residue contained in the peptide linker, the average of the number of proline residues peptide-bonded to each of the amino acid residues is preferably 0.8 or more, more preferably 1.0 or more, still more preferably 1.2 or more, still further more preferably 1.5 or more, and still further more preferably 1.8 or more. In addition, from the same viewpoint, the number of amino acid residues constituting the peptide linker may be 60 residues or more, preferably more than 60 residues, and more preferably 70 residues or more, and from the viewpoint of enhancing expression efficiency, for example, the number of amino acid residues is 600 residues or less, may be 500 residues or less, may be 400 residues or less, or may be 300 residues or less.

The α-helix linker is a rigid linker that is difficult to be bent by forming an α-helix which is a helical structure wound with 3.6 residues as a secondary structure. That is, when the peptide linker according to the peptide linker, the fusion protein, and the localization method of the present invention is the α-helix linker, an amino acid sequence constituting the peptide linker is not particularly limited as long as it can form an α-helix, and may contain a non-natural amino acid, a D-form amino acid, and the like in addition to the naturally existing L-form amino acid. When the peptide linker according to the peptide linker, the fusion protein, and the localization method of the present invention is the α-helix linker, the number of amino acid residues constituting the peptide linker may be 70 residues or more, preferably 80 residues or more, and more preferably 90 residues or more from the viewpoint of enhancing the localization of the fusion protein to a target organelle by inhibiting an intramolecular hydrophobic interaction, and from the viewpoint of enhancing the expression efficiency, for example, the number of amino acid residues is 600 residues or less, 500 residues or less, 400 residues or less, or 300 residues or less. Examples of the α-helix linker include a peptide linker having the following SEQ ID NOs: 5 to 10 as a repeating unit.
(a)EAAAK(SEQ ID NO: 5)
(b)EAAAR(SEQ ID NO: 6)
(c)EAAAQ(SEQ ID NO: 7)
(d)DAAAK(SEQ ID NO: 8)
(e)DAAAR(SEQ ID NO: 9)
(f)DAAAQ(SEQ ID NO: 10)

### (4) Fusion Protein

The fusion protein according to the peptide linker, the fusion protein, and the localization method of the present invention include at least a transmembrane protein, an organelle localization signal, and a peptide linker inserted between the transmembrane protein and the organelle localization signal. The fusion protein may be a simple protein consisting of only of an amino acid or may be a complex protein comprising a component other than the amino acid, but is usually a simple protein from the viewpoint of convenience of acquisition.

In the fusion protein according to the peptide linker, the fusion protein, and the localization method of the present invention, a linker may be further inserted between the peptide linker and the transmembrane protein and/or between the peptide linker and the organelle localization signal. The structure of the linker to be inserted therebetween is not particularly limited, but from the viewpoint of convenience of acquisition, the linker may be a linker formed by a peptide-bonded amino acid. In addition, from the viewpoint of enhancing the localization of the fusion protein to the target organelle by securing rigidity of the peptide chain between the fusion protein and the organelle localization signal and inhibiting the intramolecular hydrophobic interaction, the length of the linker may be, for example, 200 nm or less, 150 nm or less, 100 nm or less, 50 nm or less, or 25 nm or less. When the linker is a linker formed of the peptide-bonded amino acid, from the same viewpoint, the number of amino acid residues constituting the linker may be, for example, 400 residues or less, 300 residues or less, 200 residues or less, 100 residues or less, or 50 residues or less, and may be, for example, 2.0 times or less, 1.5 times or less, 1.0 times or less, or 0.5 times or less the number of amino acid residues constituting the peptide linker.

The fusion protein according to the peptide linker, the fusion protein, and the localization method of the present invention may further contain any peptide domain at a terminus to which the organelle localization signal is not bound among the peptide terminuses of the fusion protein. The peptide domain may include, for example, a fluorescent protein domain such as GFP, CFP, and RFP, and a functional domain such as an affinity tag domain such as a His tag, a FLAG tag, and a GST tag.

### (5) Nucleic Acid, Vector, and Method of Acquiring the Same

Another aspect of the present invention is a nucleic acid encoding the fusion protein according to one aspect of the present invention or a vector expressing the fusion protein according to one aspect of the present invention.

A nucleic acid according to an embodiment of the present invention is not particularly limited as long as it is a nucleic acid comprising a base sequence corresponding to the amino acid sequence of the fusion protein according to one aspect of the present invention or a base sequence having 80% or more, 85% or more, 90% or more, 95% or more, 96% or more, 97% or more, 98% or more, or 99% or more sequence identity with the base sequence, and may be deoxyribonucleic acid (DNA), ribonucleic acid (RNA), or a complex of DNA and RNA, may be a single-stranded nucleic acid or a double-stranded nucleic acid, or may be genomic DNA or cDNA (complementary DNA). A nucleic acid according to a preferred embodiment of the present invention is a nucleic acid comprising a base sequence corresponding to the amino acid sequence of the fusion protein according to one aspect of the present invention or a base sequence having 90% or more sequence identity with the base sequence.

The vector according to the embodiment of the present invention is not particularly limited as long as it can express the fusion protein according to one aspect of the present invention in a cell, and may be, for example, a transient vector or a stable expression vector, may be a plasmid comprising at least a promoter sequence and a base sequence corresponding to the amino acid sequence of the fusion protein, or may be a vector in which a base sequence corresponding to the amino acid sequence of the fusion protein is incorporated into a viral vector such as adeno-associated virus, adenovirus, retrovirus, or lentivirus. Vectors incorporating nucleic acids can include various sequences such as a restriction enzyme site, a control sequence for expression of an inserted gene, an antibiotic resistance gene, a sequence for sorting a transformant, and the like. The vector according to the embodiment of the present invention can be easily obtained by a method usually performed by a person skilled in the art, and can be obtained, for example, by introducing the nucleic acid according to the embodiment of the present invention as an insert into any vector by ligation and cloning. As a vector incorporating the above-described nucleic acid, for example, a commercially available product such as pEBMulti-Hygro vector (FUJIFILM Wako Pure Chemical Corporation) can be used.

### (6) Method of Executing Localization Method

The localization method according to one aspect of the present invention can be performed, for example, by introducing the fusion protein according to one aspect of the present invention or a vector expressing the protein into a cell. The introduction into the cell can be easily performed by a method usually performed by a person skilled in the art. For example, when the vector is a viral vector, the vector can be introduced into a cell by adding the viral vector to a culture environment of the cell or an animal individual. In addition, for example, when the vector is a plasmid or when the fusion protein itself is introduced into a cell, these can be introduced into the cell by lipofection, electroporation, microinjection, sonoporation, magnetofection, or the like.

### (7) Method of Acquiring Fusion Protein

The fusion protein according to one aspect of the present invention can be obtained, for example, by isolating and purifying the fusion protein from a host cell expressing the fusion protein by the same method as the localization method according to one aspect of the present invention. The host cell is not particularly limited, and may be a prokaryotic cell such as E. coli or Bacillus subtilis, or a eukaryotic cell such as a yeast or an animal cell. The animal cell may be, for example, a mammalian cell, and more specifically, may be a mouse cell or a human cell. In addition, the method of isolating the fusion protein from the host cell is not particularly limited, and can be performed by a method usually performed by those skilled in the art. For example, after a membrane of the host cell is solubilized, the fusion protein can be isolated and purified by a method such as dialysis, salting-out, filtration, fractionation precipitation, affinity chromatography, ion exchange chromatography, gel electrophoresis, isoelectric focusing, hydrophobic chromatography, gel filtration column chromatography, or reverse phase chromatography. These methods may be used alone, or a plurality of methods may be used in combination.

### (8) Treatment of Mitochondrial Diseases

Mitochondrial disease, which is one of congenital genetic diseases, is a generic term for diseases caused by dysfunctions of mitochondria, and is a disease that causes various serious symptoms including deterioration in muscle strength and delayed development progress. Mitochondria are the only organelles excluding nuclei that have DNA unique to an organelle called mitochondrial DNA (mtDNA) and can express a protein in the organelle. A mitochondrial protein expressed by mitochondrial DNA is essential for exerting the function of the mitochondria. In a mitochondrial disease, an abnormality in protein expression by mitochondrial DNA is observed. That is, in the mitochondrial disease, dysfunctions of mitochondria themselves occur due to deficiency or dysfunction of a protein expressed by mitochondrial DNA, resulting in various serious symptoms.

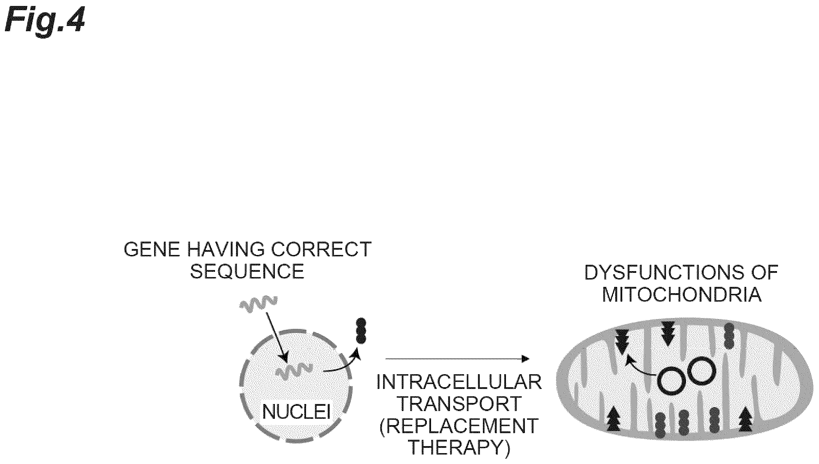

As one of the methods of treating the mitochondrial disease, as shown in FIG. 4, an approach is considered in which a protein expressed by mitochondrial DNA is expressed from nuclear DNA and is transported to mitochondria for supplementation (hereinafter, also referred to as "replacement therapy"). However, mitochondria are organelles having an inner membrane and an outer membrane which are lipid bilayer membranes, and most of mitochondrial proteins essential for life functions are transmembrane proteins. However, it has been difficult to localize transmembrane proteins in mitochondria in the related art, and thus it has been considered that treatment by replacement therapy is difficult. On the other hand, since the fusion protein or the vector according to one aspect of the present invention can localize the transmembrane protein in the target organelle in the cell, it is considered that the fusion protein or the vector can be used for replacement therapy of the mitochondrial disease.

That is, another aspect of the present invention is a therapeutic agent for a mitochondrial disease, which comprises the fusion protein or the vector according to one aspect of the present invention, in which the target organelle is mitochondria. The transmembrane protein according to the fusion protein or the vector in the present embodiment is a transmembrane protein deficient or dysfunctional in mitochondrial diseases, or a transmembrane protein capable of substituting the function of the transmembrane protein. The organelle localization signal according to the fusion protein or the vector in the present embodiment is a mitochondria targeting signal.

The fusion protein or the vector comprised in the therapeutic agent for a mitochondrial disease according to the embodiment of the present invention may be encapsulated in a drug delivery carrier such as a liposome, a microsphere, a nanosphere, a polymeric micelle, or an exosome. In addition, a method of administering the therapeutic agent for a mitochondrial disease according to the embodiment of the present invention is not particularly limited, and for example, the therapeutic agent for a mitochondrial disease can be administered by a method such as oral administration, inhalation, intravenous administration, intramuscular administration, subcutaneous administration, or transdermal administration.

### (9) Nucleic Acids Encoding Peptide Linker

Another aspect of the present disclosure is a nucleic acid comprising a sequence encoding a peptide linker according to one aspect of the present disclosure. The nucleic acid according to the embodiment of the present invention is not particularly limited as long as it is a nucleic acid comprising a base sequence corresponding to the amino acid sequence of the peptide linker according to one aspect of the present invention or a base sequence having 80% or more, 85% or more, 90% or more, 95% or more, 96% or more, 97% or more, 98% or more, or 99% or more sequence identity with the base sequence, and may be deoxyribonucleic acid (DNA), ribonucleic acid (RNA), or a complex of DNA and RNA, may be a single-stranded nucleic acid or a double-stranded nucleic acid, or may be genomic DNA or cDNA (complementary DNA). The nucleic acid according to a preferred embodiment of the present invention is a nucleic acid comprising a base sequence corresponding to the amino acid sequence of the peptide linker according to one aspect of the present invention or a base sequence having 90% or more sequence identity with the base sequence. The nucleic acid according to the embodiment of the present invention may be a nucleic acid in which a base sequence corresponding to the amino acid sequence of the peptide linker according to one aspect of the present invention or a base sequence having 80% or more, 85% or more, 90% or more, 95% or more, 96% or more, 97% or more, 98% or more, or 99% or more sequence identity with the base sequence accounts for 50% or more, 60% or more, 70% or more, 80% or more, 90% or more, 95% or more, or 100% of the base sequence of the nucleic acid.

Hereinafter, the present invention will be described in detail with reference to Examples and the like, but the present invention is not limited thereto.

### Examples

### [Experimental Method 1: Fusion Protein and Vector Used for Expression Thereof]

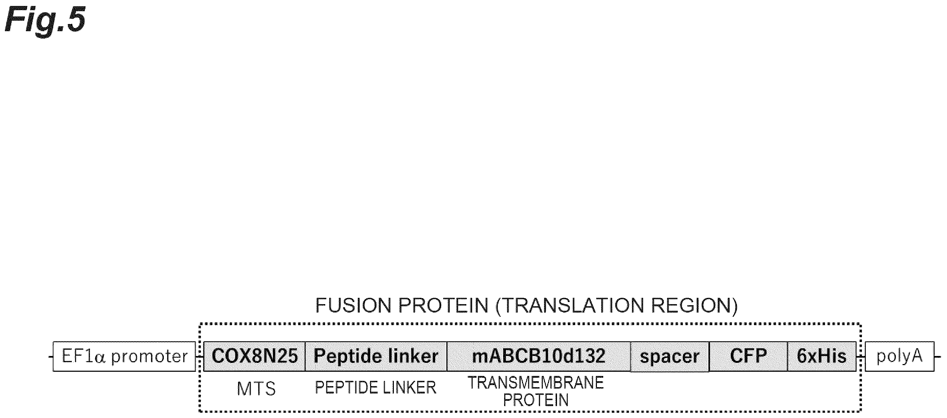

An outline of a nucleic acid corresponding to a translation region of a plasmid used for expressing a fusion protein in a cell in Examples is shown in FIG. 5. In FIG. 5, the left is a 5' terminus. The plasmid containing the translation region was prepared according to the following. A nucleic acid encoding COX8N25 and a spacer was obtained by requesting Hokkaido System Science Co., Ltd. to synthesize primers containing these sequences. A nucleic acid encoding a peptide linker was obtained by requesting Eurofins Genomics K.K. to perform the synthesis. A nucleic acid encoding CFP was purchased from the company ATUM (FPB-47-609). A nucleic acid encoding a transmembrane protein was obtained from mouse cDNA (Quick Clone cDNA, 637304, Takara Bio Inc.). These nucleic acids were PCR cloned using KOD One PCR Master Mix-Blue-(KMM-201, Toyobo Co., Ltd.), and were inserted into the pEX-A2J2 vector purchased from Eurofins Genomics K.K. using In-Fusion Snap Assembly Master Mix (Z8947N, Takara Bio Inc.), thereby producing a construct for expressing a protein. The construct was introduced into a competent cell (Competent Quick DH5a, DNA-913F) available from Toyobo Co., Ltd., was amplified, and was purified by a plasmid purification kit (NucleoSpin Plasmid, U0588A) available from Takara Bio Inc., thereby obtaining a plasmid. It is noted that a part (1615 bp) of the Chinese hamster Hipp 11 region is incorporated into the pEX-A2J2 vector, and if Cas9 protein is simultaneously expressed, it is a vector capable of knocking in by homologous recombination (Cas9 is not expressed because transient expression was sufficient in the examples described herein). The protein expressed in cells using this plasmid consists of, in order from a N-terminus, a mitochondria targeting signal (SEQ ID NO: 1, COX8N25), a peptide of extra 6 residues in cloning (glycine-serine-alanine-glycine-serine-alanine), a peptide linker, a transmembrane protein mABCB10d132 (SEQ ID NO: 11), a spacer (SEQ ID NO: 12), a fluorescent protein CFP (SEQ ID NO: 13), and a histidine tag (His tag, 6 histidine residues). The mABCB10d132 is a protein in which 132 amino acids on the N-terminus side of the mitochondrial inner membrane localization protein mouse ABCB10 are deleted, and by deleting the 132 amino acids on the N-terminus side corresponding to the mitochondria targeting signal, mitochondrial localization is lost and localization in the endoplasmic reticulum is performed (Non Patent Literature 3). Furthermore, it has been reported that localization to mitochondria is impossible even when MTS of another type of mitochondrial localized protein is added instead of the deleted N-terminus 132 amino acids (Non Patent Literature 3).

### [Experimental Method 2: Preparation of Cells Expressing Fusion Protein]

A method of preparing a cell expressing a fusion protein, which was used in Examples, is described as follows.

First, regarding the cell, a CHO-K1 cell (provided by JCRB Cell Bank, cell registration number: JCRB9018, hereinafter simply referred to as a cell) cultured in Ham's F-12 Nutrient Mix (Thermo Fisher Scientific, product number 11765054) medium (hereinafter, the medium is also simply referred to as a medium) containing a final concentration of fetal bovine serum of 10% was seeded on a 3-well dish (manufactured by AGC Techno Glass Co., Ltd., product number 3970-103) so as to be 0.7×10⁴ cells/well.

Next, regarding a solution to be used for transformation, 20 µL of Opti-MEM (Thermo Fisher Scientific, product number 31985062), 200 ng of a plasmid prepared according to Experimental Method 1, 0.2 µL of Plus reagent, and 0.8 µL of Lipofectamine LTX (Thermo Fisher Scientific, product number 15338030) were mixed, and the mixture was allowed to stand at room temperature for 25 minutes, thereby preparing a solution (transformation solution).

Finally, regarding a step of preparing a cell expressing a fusion protein, a seeded cell was cultured in a culture medium at 37°C in a 5%CO₂ atmosphere for 1 day, then 10 µL of a transformation solution was added per 1 well to the culture medium in which the cell was cultured, and the mixture was allowed to stand at 37°C in a 5%CO₂ atmosphere for 4 to 5 hours. Thereafter, the medium was replaced with a medium not containing the transformation solution, and the cell was further cultured for 1 day at 37°C in a 5%CO₂ atmosphere.

### [Experimental Method 3: Fluorescent Staining of Mitochondria]

After a lapse of 24 hours from the addition of the transformation solution, the cell expressing the fusion protein prepared in Experimental Method 2 was cultured in a medium in which Mitotracker Deep Red (Thermo Fisher Scientific, product number M22426, hereinafter also referred to as MTDR) having a final concentration of 50nM was dissolved at 37°C in a 5%CO₂ atmosphere for 1 hour, thereby fluorescently staining mitochondria.

### [Experimental Method 4: Fluorescence Microscopic Observation]

The cell obtained in Experimental Method 3 was subjected to fluorescence microscopic observation according to the following protocol. 1 to 6 hours after the fluorescence staining of mitochondria, the cell was subjected to fluorescence microscopic observation using an electric inverted microscope IX83 (manufactured by Olympus Corporation) equipped with a 100 × objective lens UplanSApo 100x (manufactured by Olympus Corporation) and a digital CMOS camera ORCA-flash4, 0V3 (C13440-20CU manufactured by Hamamatsu Photonics K.K.), and a fluorescence image was acquired. As a fluorescent mirror unit, X3-FGWXL (excitation wavelength: 530 to 550 nm, fluorescence wavelength: 570 nm or more) was used for fluorescence observation of MTDR (mitochondria), and IX3-FCFPXL (excitation wavelength: 425 to 445 nm, fluorescence wavelength: 460 to 510 nm) was used for fluorescence observation of CFP (fusion protein).

### [Experimental Method 5: Co-localization Analysis]

The fluorescence images of MTDR and CFP obtained in Experimental Method 4 were subjected to co-localization analysis according to the following protocol. Regarding the fluorescence image, an image from which high-frequency components including noise were removed by a median filter having a window size of 35 pixels (pixel resolution of 130 nm/pixel) was created, and subtraction was performed from an original fluorescence image to create an analysis image from which background light was removed from the fluorescence image. Regarding two images for analysis obtained from the fluorescence images of MTDR and CFP, a correlation coefficient Pearson Correlation Coefficient (hereinafter, also simply referred to as a "correlation coefficient") of a luminance value was calculated according to a method described in a known literature (Costes SV et al., Biophys. J. 86, 3993-4003 (2004)).

A significant difference test of a correlation coefficient between different groups was performed by two-tailed test (Mann-Whitney U test) with 1% significance.

### [Example 1: Relationship Between Number of Amino Acid Residues Contained in Proline Linker and Localization Effect]

When the peptide linker is a proline linker, a relationship between the number of amino acid residues contained in the proline linker and a localization effect was examined.

In accordance with Experimental Methods 1 to 5, cells expressing a fusion protein in which the number of amino acid residues contained in the proline linker was 0, 50, 60, 70, 80, 90, 100, 150, or 200 residues and 50% of the amino acid residues contained in the proline linker were proline residues were subjected to fluorescence microscopic observation and co-localization analysis. As an example, the amino acid sequence of the expressed fusion protein when the number of amino acid residues contained in the proline linker is 100 residues is shown in SEQ ID NO: 14, and the base sequence of the nucleic acid corresponding to a translation region contained in the plasmid used to express the fusion protein is shown in SEQ ID NO: 15. When the number of amino acid residues contained in the proline linker was less than 100 residues, the number of amino acid residues corresponding to the number of amino acid residues contained in each group of linkers from the C-terminus side of SEQ ID NO: 16 was used as a proline linker region, instead of a proline linker region 100 residues (SEQ ID NO: 16) contained in the amino acid sequence of the fusion protein shown in SEQ ID NO: 14. When the number of amino acid residues contained in the proline linker was 150 residues, an amino acid sequence further having 50 residues on the N-terminus side of SEQ ID NO: 16 on the N-terminal side in the amino acid sequence shown in SEQ ID NO: 16 was used as a proline linker region, instead of the proline linker region 100 residues (SEQ ID NO: 16) contained in the amino acid sequence of the fusion protein shown in SEQ ID NO: 14. When the number of amino acid residues contained in the proline linker was 200 residues, an amino acid sequence further having the amino acid sequence shown in SEQ ID NO: 16 on the N-terminus side in the amino acid sequence shown in SEQ ID NO: 16 was used as a proline linker region, instead of the proline linker region 100 residues (SEQ ID NO: 16) contained in the amino acid sequence of the fusion protein shown in SEQ ID NO: 14. In addition, the fusion protein in which the number of amino acid residues contained in the proline linker is 0 is a fusion protein having no peptide linker region, and the plasmid used to express the fusion protein is also a plasmid having no region encoding the peptide linker.

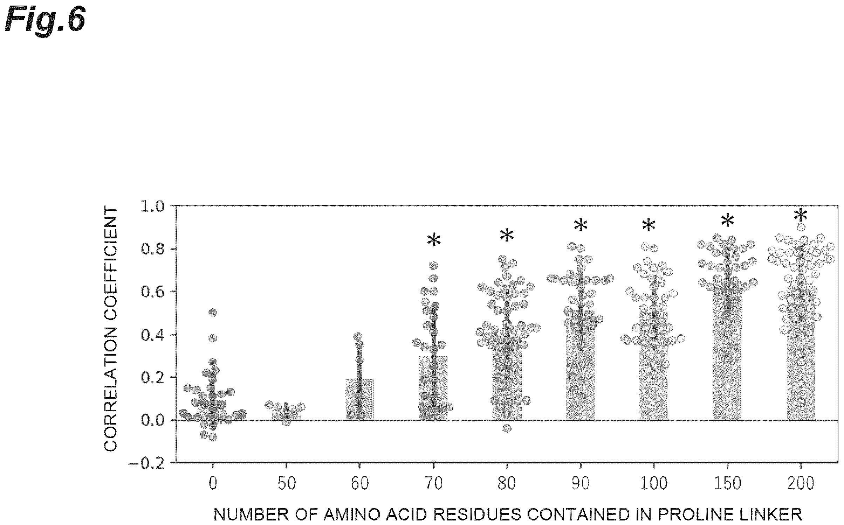

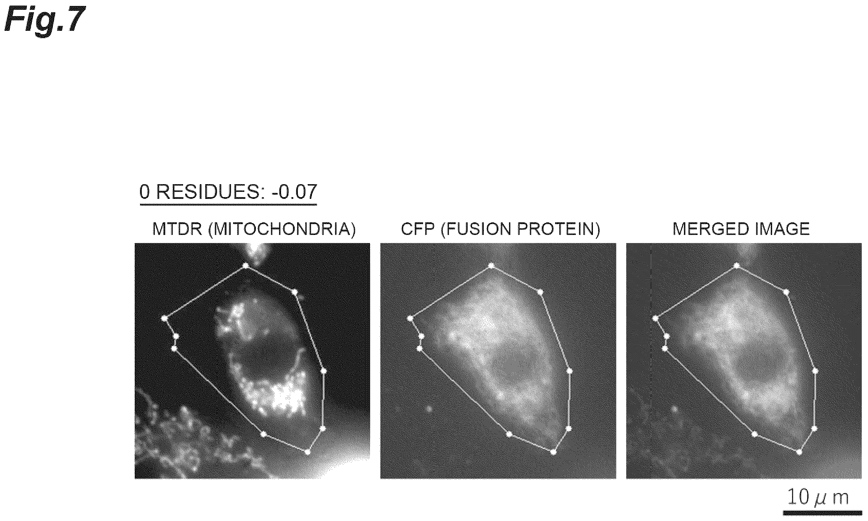

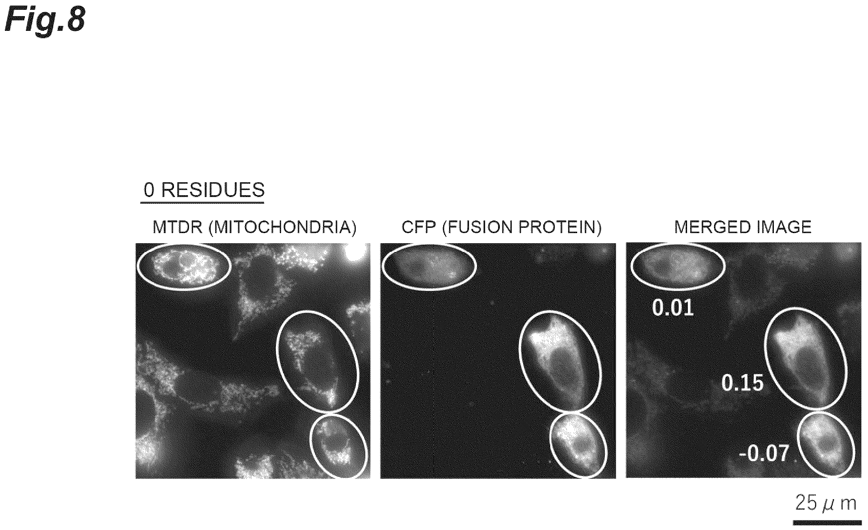

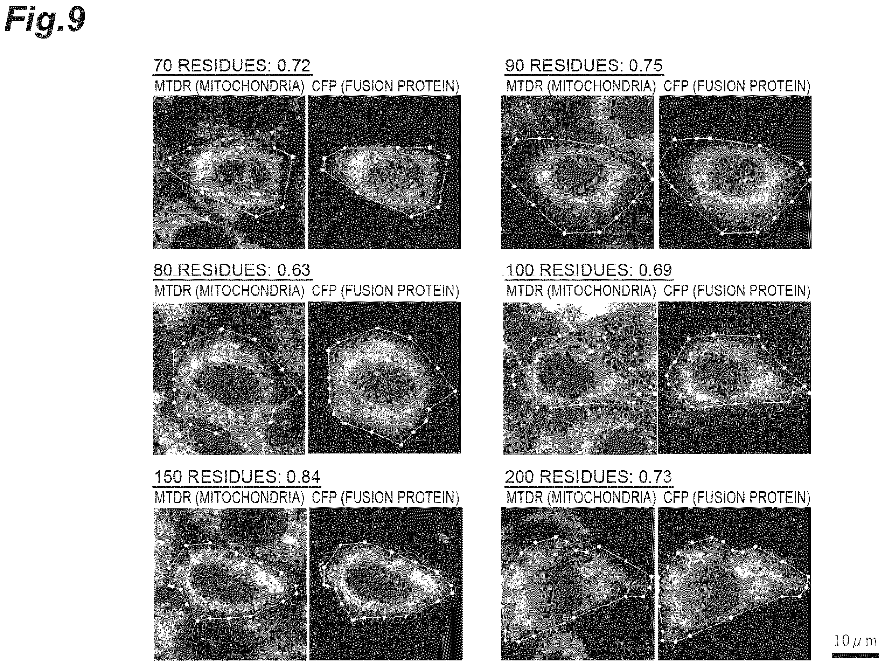

A correlation coefficient in the co-localization analysis according to the number of amino acid residues contained in the proline linker is shown in FIG. 6. In FIG. 6, a gray bar graph indicates an average value, a black error bar indicates a standard deviation, and an asterisk (*) indicates that there is a significant difference in a correlation coefficient between the number of amino acid residues and the group of 0 residues. In addition, representative fluorescence images in the group of 0 amino acid residues are shown in FIGS. 7 and 8, and representative fluorescence images in the group of 70, 80, 90, 100, 150, and 200 residues are shown in FIG. 9. In FIGS. 7 to 9, numerical values described beside the number of amino acid residues or in the vicinity of the outside of a white line indicate correlation coefficients in a region surrounded by the white line for each image.

As shown in FIG. 6, in the fusion protein in which the number of amino acid residues contained in the proline linker was 60 residues or more, an increase in the average value of the correlation coefficient was observed, and in the fusion protein in which the number of amino acid residues was 70 residues or more, a difference in the correlation coefficient was significant between the fusion protein and the fusion protein in which the number of amino acid residues was 0 residues. In addition, as shown in FIGS. 7 and 8, it was strongly suggested that fusion proteins having 0 amino acid residues were distributed in a mesh shape in the entire cell and were migrated to the endoplasmic reticulum, whereas as shown in FIG. 9, fusion proteins having 70, 80, 90, 100, 150, and 200 amino acid residues gave a fluorescence image very similar to MTDR and were strongly suggested to be localized in mitochondria.

### [Example 2: Relationship Between Proportion of Proline Residues Contained in Proline Linker and Localization Effect]

When the peptide linker is a proline linker, a relationship between the proportion of proline residues contained in the proline linker and the localization effect was examined.

According to Experimental Methods 1 to 5, cells expressing a fusion protein in which the number of amino acid residues contained in the proline linker was 100 residues and 30%, 40%, or 50% of the amino acid residues were proline residues were subjected to fluorescence microscopic observation and co-localization analysis. When 30% of the amino acid residues contained in the proline linker are proline residues, the amino acid sequence of the expressed fusion protein is shown in SEQ ID NO: 17, and the base sequence of the nucleic acid corresponding to the translation region contained in the plasmid used to express the fusion protein is shown in SEQ ID NO: 18. When 40% of the amino acid residues contained in the proline linker are proline residues, the amino acid sequence of the expressed fusion protein is shown in SEQ ID NO: 19, and the base sequence of the nucleic acid corresponding to the translation region contained in the plasmid used to express the fusion protein is shown in SEQ ID NO: 20. When 50% of the amino acid residues contained in the proline linker are proline residues, the amino acid sequence of the expressed fusion protein is the same as the amino acid sequence shown in SEQ ID NO: 14, and the base sequence of the nucleic acid corresponding to the translation region contained in the plasmid used to express the fusion protein is the same as the base sequence shown in SEQ ID NO: 15.

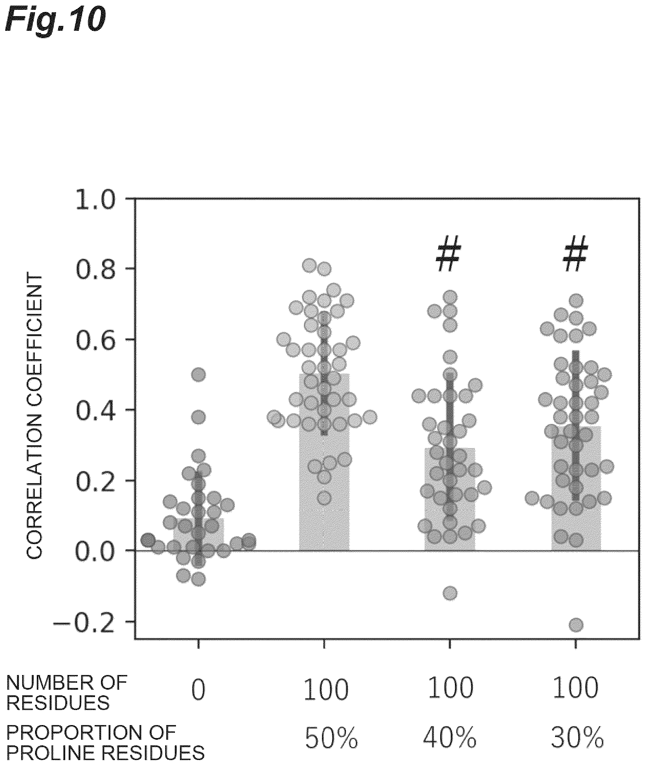

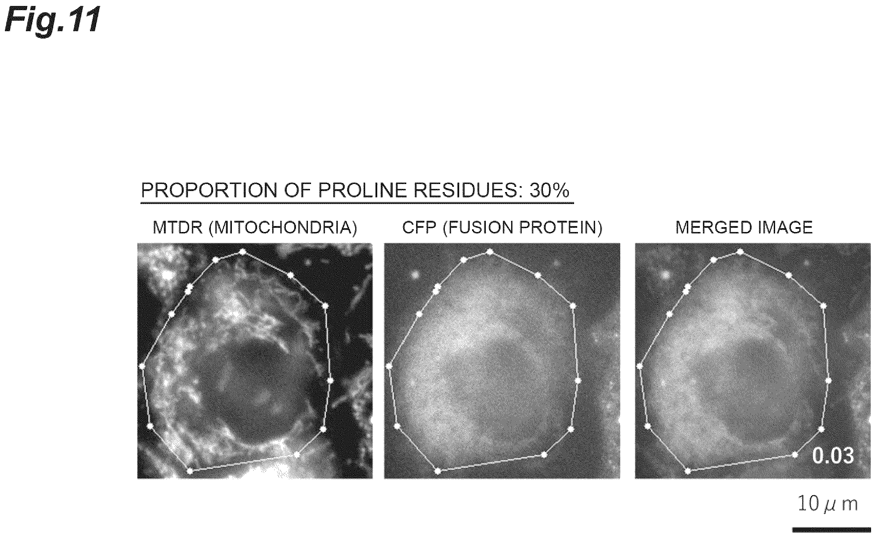

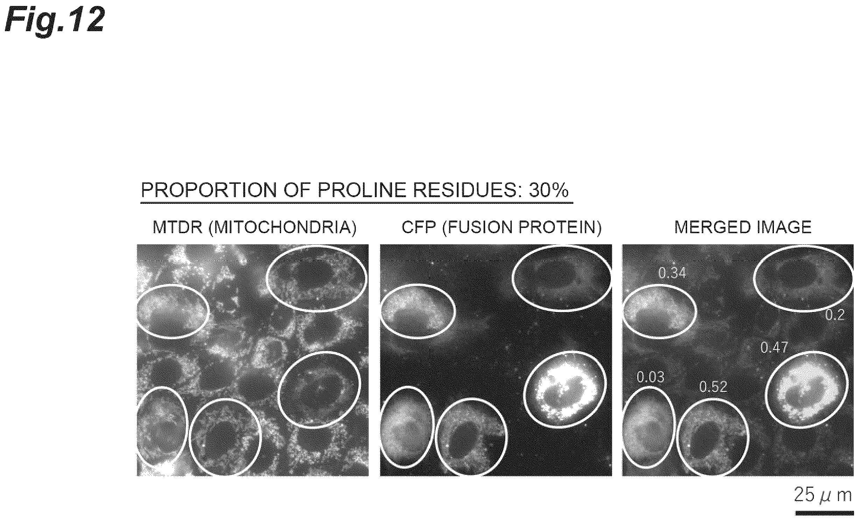

The correlation coefficient in the co-localization analysis according to the proportion of the proline residue in the amino acid residues contained in the proline linker is shown in FIG. 10. In FIG. 10, a gray bar graph indicates a mean value, a black error bar indicates a standard deviation, and a sharp mark (#) indicates that there is a significant difference in the correlation coefficient in the case of a group in which 50% of the amino acid residues are proline residues. Representative fluorescence images in a group in which 30% of the amino acid residues are proline residues are shown in FIGS. 11 and 12. In FIGS. 11 and 12, numerical values described in the vicinity of the outside of a white line indicate correlation coefficients in a region surrounded by the white line for each image.

As shown in FIG. 10, a correlation coefficient decreased in the fusion protein in which the proportion of the proline residues to the amino acid residues contained in the proline linker is 30% and 40%, and a difference in correlation coefficient was significant between the previous correlation coefficient and a correlation coefficient in the case of the fusion protein in which the proportion of the proline residues to the amino acid residues contained in the proline linker is 50%. In addition, as shown in FIGS. 11 and 12, it was strongly suggested that the fusion protein in which the proportion of proline residues to the amino acid residues contained in the proline linker was 30% was distributed in a mesh shape in the entire cell and was transferred to the endoplasmic reticulum, similarly to the fusion protein having no proline linker region. A similar tendency was observed in the fusion protein in which the proportion of proline residues thereto is 40%.

### [Example 3: Relationship Between Number of Amino Acid Residues Contained in α-Helix Linker and Localization Effect]

When the peptide linker is an α-helix linker, a relationship between the number of amino acid residues contained in the α-helix linker and a localization effect was examined.

According to Experimental Methods 1 to 5, cells expressing a fusion protein in which the number of amino acid residues contained in the α-helix linker was 0, 50, 60, 70, 80, 90, 100, 200, 300, 400, 500, or 600 residues were subjected to fluorescence microscopic observation and co-localization analysis. The amino acid sequence of the expressed fusion protein when the number of amino acid residues contained in the α-helix linker is 50 residues is shown in SEQ ID NO: 21, and the base sequence of the nucleic acid corresponding to the translation region contained in the plasmid used to express the fusion protein is shown in SEQ ID NO: 22. The amino acid sequence of the expressed fusion protein when the number of amino acid residues contained in the α-helix linker is 60 residues is shown in SEQ ID NO: 23, and the base sequence of the nucleic acid corresponding to the translation region contained in the plasmid used to express the fusion protein is shown in SEQ ID NO: 24. The amino acid sequence of the expressed fusion protein when the number of amino acid residues contained in the α-helix linker is 70 residues is shown in SEQ ID NO: 25, and the base sequence of the nucleic acid corresponding to the translation region contained in the plasmid used to express the fusion protein is shown in SEQ ID NO: 26. The amino acid sequence of the expressed fusion protein when the number of amino acid residues contained in the α-helix linker is 80 residues is shown in SEQ ID NO: 27, and the base sequence of the nucleic acid corresponding to the translation region contained in the plasmid used to express the fusion protein is shown in SEQ ID NO: 28. The amino acid sequence of the expressed fusion protein when the number of amino acid residues contained in the α-helix linker is 90 residues is shown in SEQ ID NO: 29, and the base sequence of the nucleic acid corresponding to the translation region contained in the plasmid used to express the fusion protein is shown in SEQ ID NO: 30. The amino acid sequence of the expressed fusion protein when the number of amino acid residues contained in the α-helix linker is 100 residues is shown in SEQ ID NO: 31, and the base sequence of the nucleic acid corresponding to the translation region contained in the plasmid used to express the fusion protein is shown in SEQ ID NO: 32. The amino acid sequence of the expressed fusion protein when the number of amino acid residues contained in the α-helix linker is 200 residues is shown in SEQ ID NO: 33, and the base sequence of the nucleic acid corresponding to the translation region contained in the plasmid used to express the fusion protein is shown in SEQ ID NO: 34. The amino acid sequence of the expressed fusion protein when the number of amino acid residues contained in the α-helix linker is 300 residues is shown in SEQ ID NO: 35, and the base sequence of the nucleic acid corresponding to the translation region contained in the plasmid used to express the fusion protein is shown in SEQ ID NO: 36. The amino acid sequence of the expressed fusion protein when the number of amino acid residues contained in the α-helix linker is 400 residues is shown in SEQ ID NO: 37, and the base sequence of the nucleic acid corresponding to the translation region contained in the plasmid used to express the fusion protein is shown in SEQ ID NO: 38. The amino acid sequence of the expressed fusion protein when the number of amino acid residues contained in the α-helix linker is 500 residues is shown in SEQ ID NO: 39, and the base sequence of the nucleic acid corresponding to the translation region contained in the plasmid used to express the fusion protein is shown in SEQ ID NO: 40. The amino acid sequence of the expressed fusion protein when the number of amino acid residues contained in the α-helix linker is 600 residues is shown in SEQ ID NO: 41, and the base sequence of the nucleic acid corresponding to the translation region contained in the plasmid used to express the fusion protein is shown in SEQ ID NO: 42. In addition, a fusion protein in which the number of amino acid residues contained in the α-helix linker is 0 and a plasmid used for expressing the fusion protein are the same as the fusion protein in which the number of amino acid residues contained in the linker used in Example 1 is 0 and the plasmid used for expressing the fusion protein.

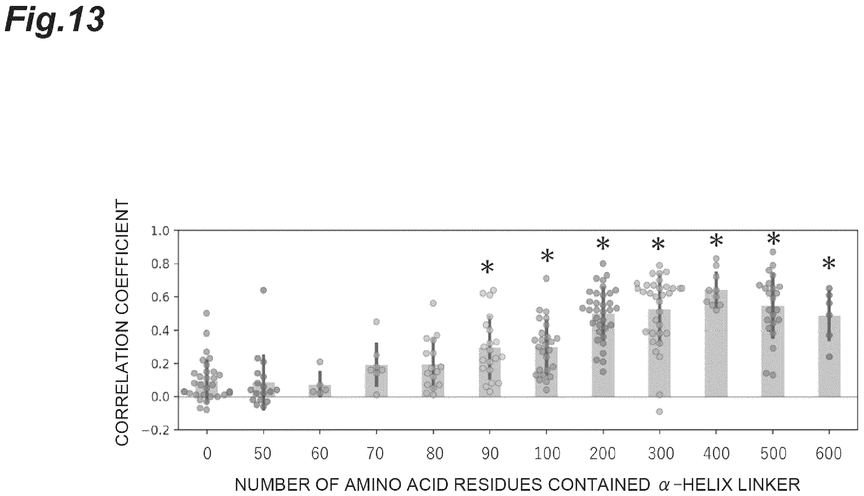

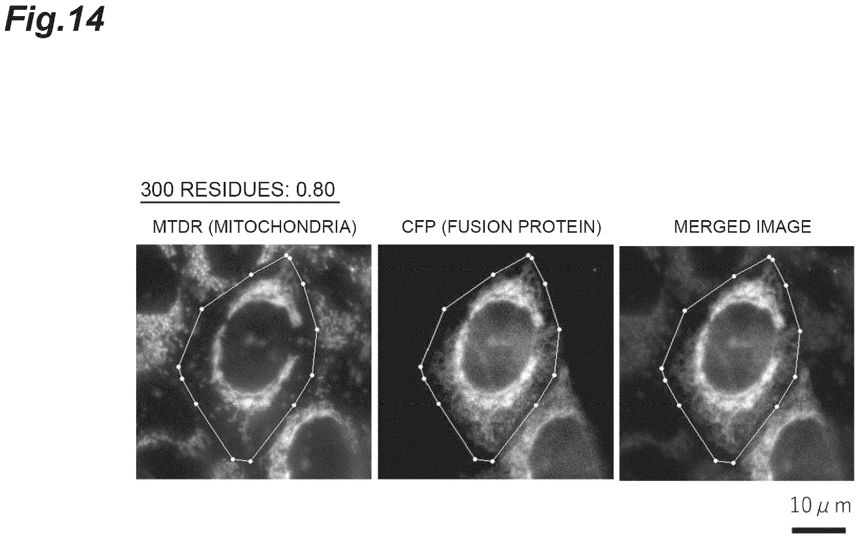

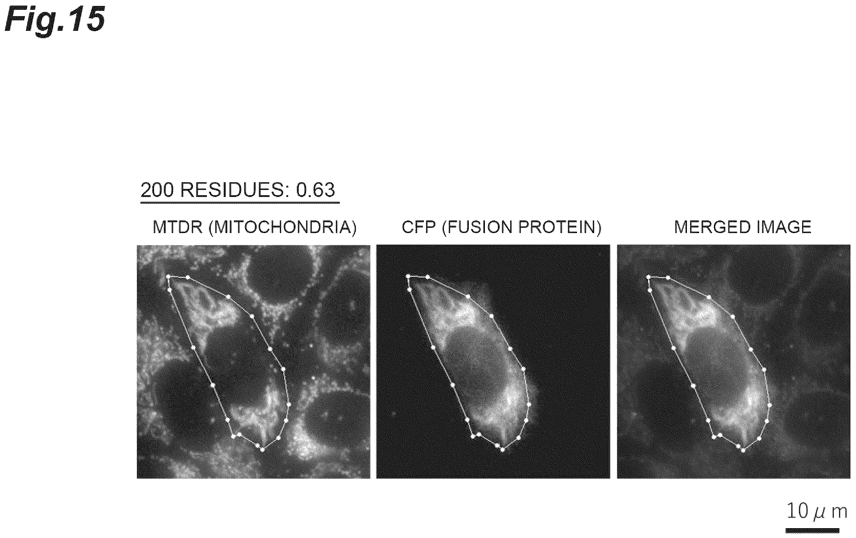

A correlation coefficient in the co-localization analysis according to the number of amino acid residues contained in the α-helix linker is shown in FIG. 13. In FIG. 13, a gray bar graph indicates an average value, a black error bar indicates a standard deviation, and an asterisk (*) indicates that there is a significant difference in a correlation coefficient between the number of amino acid residues and the group of 0 residues. In addition, representative fluorescence images in the group of 300 amino acid residues are shown in FIG. 14, and a representative fluorescence image in the group of 200 residues is shown in FIG. 15. In FIGS. 14 and 15, a numerical value described next to the number of amino acid residues indicates a correlation coefficient in a region surrounded by a white line for each image.

As shown in FIG. 13, in the fusion protein in which the number of amino acid residues contained in the α-helix linker was 70 residues or more, an increase in the average value of the correlation coefficient was observed, and in the fusion protein in which the number of amino acid residues was 90 residues or more, a difference in the correlation coefficient was significant between the fusion protein and the fusion protein in which the number of amino acid residues was 0 residues. In addition, as shown in FIGS. 14 and 15, the fusion proteins having 200 and 300 amino acid residues gave fluorescence images well similar to MTDR, strongly suggesting localization in mitochondria.

## Claims

1. A peptide linker for localizing a transmembrane protein to a target organelle, the peptide linker being inserted between the transmembrane protein and an organelle localization signal corresponding to the target organelle, wherein:
the number of proline residues contained in the peptide linker exceeds 40% of the number of all amino acid residues contained in the peptide linker, and
the number of amino acid residues constituting the peptide linker is 60 residues or more.

2. A peptide linker for localizing a transmembrane protein to a target organelle, the peptide linker being inserted between the transmembrane protein and an organelle localization signal corresponding to the target organelle, wherein:
the peptide linker is formed of a peptide of which the secondary structure is an α-helix, and
the number of amino acid residues constituting the peptide linker is 70 residues or more.

3. A fusion protein comprising:
a transmembrane protein;
an organelle localization signal for localization to a target organelle; and
a peptide linker inserted between the transmembrane protein and the organelle localization signal, wherein:
the number of proline residues contained in the peptide linker exceeds 40% of the number of all amino acid residues contained in the peptide linker, and
the number of amino acid residues constituting the peptide linker is 60 residues or more.

4. A fusion protein comprising:
a transmembrane protein;
an organelle localization signal for localization to a target organelle; and
a peptide linker inserted between the transmembrane protein and the organelle localization signal, wherein:
the peptide linker is formed of a peptide of which the secondary structure is an α-helix, and
the number of amino acid residues constituting the peptide linker is 70 residues or more.

5. A method of localizing a transmembrane protein to a target organelle, the method comprising inserting a peptide linker between the transmembrane protein and an organelle localization signal for localization to the target organelle, wherein:
the number of proline residues contained in the peptide linker exceeds 40% of the number of all amino acid residues contained in the peptide linker, and
the number of amino acid residues constituting the peptide linker is 60 residues or more.

6. A method of localizing a transmembrane protein to a target organelle, the method comprising inserting a peptide linker between the transmembrane protein and an organelle localization signal for localization to the target organelle, wherein:
the peptide linker is formed of a peptide of which the secondary structure is an α-helix, and
the number of amino acid residues constituting the peptide linker is 70 residues or more.

7. A nucleic acid encoding the fusion protein according to claim 3 or 4.

8. A vector expressing the fusion protein according to claim 3 or 4.

9. A therapeutic agent for a mitochondrial disease, comprising a vector expressing the fusion protein according to claim 3 or 4 or the fusion protein according to claim 3 or 4, wherein the target organelle is mitochondria.

10. A nucleic acid comprising a sequence encoding the peptide linker according to claim 1 or 2.
